# EUROPEAN PATENT SPECIFICATION

(11) **EP 2 448 826 B1**
(45) Date of publication and mention of the grant of the patent: **26.11.2014**
(21) Application number: 10794440.7
(22) Date of filing: 24.06.2010
(51) Int. Cl.: B65B 31/02, B65B 55/02, B65B 55/10, B65B 43/52, B65B 43/54, A61L 2/20

(54) **A DEVICE AND A METHOD FOR MAINTAINING A GAS FLOW BARRIER BETWEEN TWO VOLUMES OF A CHANNEL**
VORRICHTUNG UND VERFAHREN ZUR AUFRECHTERHALTUNG EINER GASFLUSSBARRIERE ZWISCHEN ZWEI VOLUMEN EINES KANALS
DISPOSITIF ET PROCEDE POUR MAINTENIR UNE BARRIERE D'ECOULEMENT DE GAZ ENTRE DEUX VOLUMES D'UN CONDUIT

(30) Priority: 03.07.2009 SE 0900913
(43) Date of publication of application: 09.05.2012
(73) Proprietor: Tetra Laval Holdings & Finance S.A., 1009 Pully (CH)
(72) Inventor: APPARUTI, Daniele, 41050 Montale Rangone (IT); LINDBLAD, Ulf, 224 56 Lund (SE); OLSSON, Jenny, 237 33 Bjärred (SE); OLSSON, Michael, 211 48 Malmö (SE); OMRANE, Alaa, 226 55 Lund (SE)
(74) Representative: Tetra Pak - Patent Attorneys SE
(86) International application number: PCT/SE2010/000179
(87) International publication number: WO 2011/002382

(56) References cited:
- WO-A1-2004/054883
- WO-A1-2007/036492
- DE-A1-102006 036 763
- US-A1- 2006 231 157
- US-B1- 6 691 747
- US-B1- 6 691 747

## Description

### Technical Field

The present invention relates to a method and a device for maintaining a gas flow barrier between two interconnected volumes. In particular the present invention relates to separation between two volumes having atmospheres with different degrees of sterilization, the volumes being parts of a filling machine for filling preformed packaging containers with food products.

### Technical Background

In the context mentioned above the preformed packaging containers are processed in a filling machine. The preformed packaging containers may be of the type commonly referred to as ready-to-fill packaging containers, which will be called "packages" in the following. The packages have a tubular body, generally formed from a packaging laminate having a paper core. At one end the tubular body is provided with shoulders and an opening device, such as a screw cap in a conventional manner. The opposing end is left open during production of the package, and as the package is arranged in a filling machine it is ready to be filled through the open end thereof. In the filling machine the packages are carried on carrier means, held by their closed end, and transported in a machine direction through channel, thus passing a preheating zone, a sterilization zone, a venting zone, and a filling zone where the packages are filled and sealed. The sterilization is commonly effected by means of a gas phase sterilizing agent, such as hydrogen peroxide mixed with air, and in order to avoid condensation of sterilizing agent onto surfaces of the package, the package is preheated prior to sterilization. In the venting zone the interior of the package is flushed with a venting gas (usually sterile, filtered air) for removal of sterilizing agent residues.

The term sterilization is taken to signify in the following disclosure that the package, after sterilization, attains a level of sterilization which is designated commercially sterile. It is apparent that the level of sterilization is determined by the properties during sterilization and by the properties of the atmosphere to which the interior of the package is subjected prior to being sealed. The adequate sterile conditions thus need to be maintained throughout the processing steps following the sterilization.

As mentioned above the packages are transported through the process on a transport arrangement having carrier means for carrying the packages by their closed end, and starting with the sterilization step the interior of the package needs to be kept under aseptic conditions until the package has been sealed. The filling machine may generally be an intermittent machine in which packages are transported forward from one station to the next, however, the invention, as it will be presented in the following, may also be used in a machine having a continuous flow of packages.

An apparatus of the above kind, and a corresponding method for producing and sterilizing and filling package which is referable to this context, is disclosed in published international Application WO2004/054883. In that particular application two commonly used approaches for maintaining sterile conditions are disclosed;
1) maintenance of a higher pressure in a sterilization zone than in surrounding zones, so as to avoid introduction of contaminated air into the sterilization zone;
2) arrangement of a unidirectional flow of sterilization agent in the direction from the open end of the packaging container towards the closed end of the same, so as to avoid recontamination of the interior of the packaging container. For this purpose the sterilization zone of this prior art device comprises means for controlling the flow of gaseous sterilization agent in a top portion of the sterilization zone, and means to evacuate the sterilization agent in a lower portion of the sterilization zone.

In the sterilization zone there are two main issues that have to be considered. First of all the interior of the package needs to be sterilized, which may be performed with the gas-phase sterilizing agent. Secondly reinfection of the interior of the package needs to be prevented, that is there must be no flow or transport of non-sterile gases or particles into the package following the sterilization stage. This involves e.g. migration of particles from a non-sterile portion of the outer surface of the package and entrainment of non-sterile gases with the flow of sterilizing gas or venting gas. One solution to this problem could be to use sterilizing gas in a co-flow too, such that entrainment of gas would result in entrainment of sterilizing agent, which would not affect the sterility of the package. However, this approach would probably incur an excessive consumption of sterilizing agent to be economically viable presently, and in zones subsequent (downstream in the machine direction) the sterilization zone the maintenance of aseptic conditions may not be achieved by using sterilizing gas. Though being functional, the creation of a unidirectional flow requires large mass flows of air, which necessitates a corresponding high capacity of auxiliary equipment, such as fans and filters etc. The low-velocity flow is in practice effected by ejecting the air through large perforated plates, which may have to be cleaned manually when the machine is cleaned. This is obviously labor intensive.

Also, the low-velocity flow may be sensitive to flow disturbances, which implies that the flow pattern in neighboring zones needs to be controlled, beyond what is practically feasible. Also the perforated plates are highly complex and expensive.

Thus, it is apparent that there is room for an alternative and in some aspects improved device and method for maintaining a gas flow barrier between two interconnected volumes.

US 6, 691, 747 discloses a gassing rail using a venturi effect.

### Summary

The present invention addresses the above problems by means of a device in accordance with claim 1, and a corresponding method in accordance with claim 5. Additional embodiments are defined in the subclaims.

The invention thus provides a device for maintaining, in a filling machine, a gas flow barrier between two volumes of a channel, said channel being adapted for transportation of packages in a length direction thereof, and said volumes comprising a first volume having a first degree of sterilization, and a second volume having a second degree of sterilization, wherein
- the first volume comprises gas injection means,
- the second volume comprises gas evacuation means,
- the first and second volume meet in an interface area extending in a length direction of the channel. The device is characterized in that the two volumes meet in venturi zone, a portion of the channel having a reduced cross section defined by structured indentations extending in the length direction of the channel. At the interface area, the flow is directed from the upstream zone with a higher degree of sterilization to the downstream zone, with a lower degree of sterilization. This downstream zone may contain the carrier means and part of the package, as will be discussed in the detailed description.

The construction of the inventive device results in a flow from the first volume to the second volume, exactly as in prior art. With the inventive device, however, the flow restriction caused by the reduced cross section will result in an accelerated flow rate in that particular region of the device. This flow having a higher flow rate will be less sensitive to interference from potentially disturbing flows. The effect is that a reliable gas flow barrier will be established in the interface region between the first and the second volume, preventing gas or particles to be transported from the second volume to the first. Moreover, this effective gas flow barrier does not require an excessive flow of gas from the gas injection means. In the sterilization zone the flow from the sterilization nozzle may suffice, and in the venting zone the flow from the venting nozzle may suffice, etc. In case of sterilization this implies that the sterilization agent will not be diluted, and that the concentration of sterilization agent will increase in the first volume. This will in turn increase its effects on the outside of a package being sterilized, and increase the degree of sterilization in the first volume on a hole. The flow restriction will be even more pronounced when a package is arranged in the channel, thus blocking part of the region having an already reduced cross section. Another risk of reinfection occurs as the packages are transported in the machine direction. This transportation may, and will in most case, cause a wake on the side of the package opposing the transportation direction. The wake is a volume of lower pressure, which may result in that less sterile gas, or non-sterile gas, is sucked into the volume from below. The gas may then be entrained into the package by processing jets (such as venting air).

According to one or more embodiments the gas injection means comprise a nozzle arranged to direct a gas flow into a package and receive the flow returning from the package, and redirecting the flow essentially parallel to the outside of the package, in the direction from the first volume towards the second. The use of such a nozzle will efficiently direct the flow in the first volume downwards, towards the second volume, which will facilitate the establishment of a gas flow barrier. By doing so, the flow rate may be reduced even further without compromising the gas flow barrier. The nozzle preferably injects gas into the package through several nozzle openings having an inclination relative to a central axis of the package. This will result in a downwardly directed swirling flow along the inner circumference of the package. The swirling flow will cause a reduced pressure along the central axis of the package, and thus the return flow will follow the central axis. The nozzle may then collect the return flow and redirect it by leading it through orifices in its interior.

In one or more embodiments the device may further comprise carrier means for transportation of the packages by their closed end through the channel, wherein the carrier means comprise flow directors, for directing the gas flow. The flow directors have the greatest impact when a package is not carried by the carrier comprising the flow director. The flow director will then direct and stabilize the flow, so that a stable gas barrier may be maintained. This may be considered a simple task, but one should then bear in mind the complexity of the flow for the specific nozzle that may be used. The nozzle is designed to create a high velocity, swirling flow on the inside of the package. When a package is not present, this flow will be directed directly towards the venturi zone, where it will cause a flow which is very much different from the case when a package is present.

The flow directors may extend into the venturi zone for reduction of the cross section of this zone. This will further increase the effect of increasing the flow rate in the venturi zone.

The inventive method for maintaining, in a filling machine, a gas flow barrier between two volumes of a channel, said channel being adapted for transportation of packages in a length direction thereof, and said volumes comprising a first volume having a first degree of sterilization, and a second volume having a second degree of sterilization, wherein
- the first volume comprises gas injection means,
- the second volume comprises gas evacuation means,
- the first and second volume meet in an interface area extending in a length direction of the channel,
and is characterized in the step of guiding the flow from the first volume to the second through a venturi zone, a portion of the channel having a reduced cross section, in which the two volumes meet, the reduced cross section being defined by structrural indentations extending in the length direction of the channel.

Similar to the device the gas injection means comprise a nozzle, the method may further comprising the step of directing a gas flow from the nozzle into a package, receiving the flow returning from the package with the nozzle, and redirecting, by means of the nozzle, the flow essentially parallel to the outside of the package, in the direction from the first volume towards the second. Further the carrier means may be arranged for transportation of the packages by their closed end through the channel, wherein the carrier means comprise flow directors, for directing the gas flow, and the flow directors may extend into the venturi zone for reduction of the cross section of this zone. These features results in the same advantages as for the inventive device. Though features are mentioned in combination they may be used separately.

### Brief Description of the Drawings

Fig. 1 is a schematic perspective view, partly in cross section, of a prior art filling machine used for filling of preformed packages having one open end.
Fig. 2 is a cross sectional view of a prior art gas injection nozzle, which is advantageously used in connection to the present invention
Fig. 3 is a schematic sectional view, orthogonal to a transportation direction, of a filling machine according to a first embodiment.
Figs 4A and B is a side view and a plan view, respectively of a carrier according to one embodiment of the invention.
Fig. 5 corresponds to the cross section of Fig. 3, without a package arranged in the carrier.
Figs 6 and 7 correspond to another application of one embodiment of the present invention.

### Detailed Description

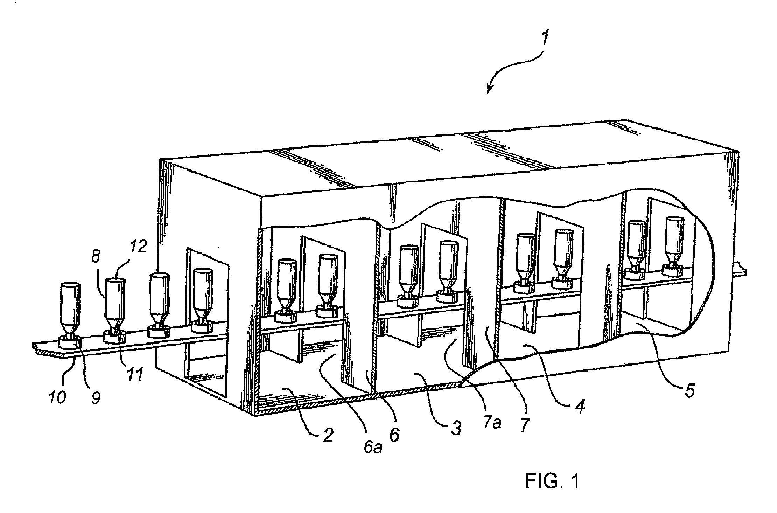

Fig. 1 illustrates a prior art filling machine, as disclosed in the previously mentioned application WO2004/054883. The device 1 has a heating zone 2, a sterilization zone 3, a venting zone 4, and connected thereto a filling zone 5. As may be seen in Fig. 1, the zones 2, 5 are separated from each other by partitionings 6, 7. The partitionings are located between two adjacent packages 8 so that a discrete number of packages 8 are located in each zone. In each partitioning 6, 7 there is an opening 6a, 7a. Packages 8 are arranged in holders 9 on a conveyor belt 10 which passes through the zones 2, 5. The packages 8 stand on their closed top end 11 with their open bottom end 12 directed upwards.

In the heating zone 2 there is a nozzle arrangement (not shown) in a top portion thereof for introduction of hot filtered air. In a bottom portion of the heating zone 2 there are outlets (not shown) for withdrawing the hot air.

Similarly, there are nozzles (not shown) for introduction of gaseous hydrogen peroxide in a top portion of the sterilization zone 3. In a bottom portion of the sterilization zone there are outlets (not shown) for withdrawing hydrogen peroxide.

The venting zone 4 also has nozzles (not shown) for introducing hot sterile air in a top portion. In a bottom portion of the venting zone 4 there are outlets (not shown) for withdrawing hot air.

In a manner similar to the heating, sterilization and venting zones 2-4, the filling zone 5 has nozzles 26 for introducing sterile air in a top portion 27 of the filling zone.

The filling machine also has a gas production unit for producing the gaseous hydrogen peroxide used for sterilization, as well as a catalyst unit for degrading hydrogen peroxide gas withdrawn from the sterilization zone.

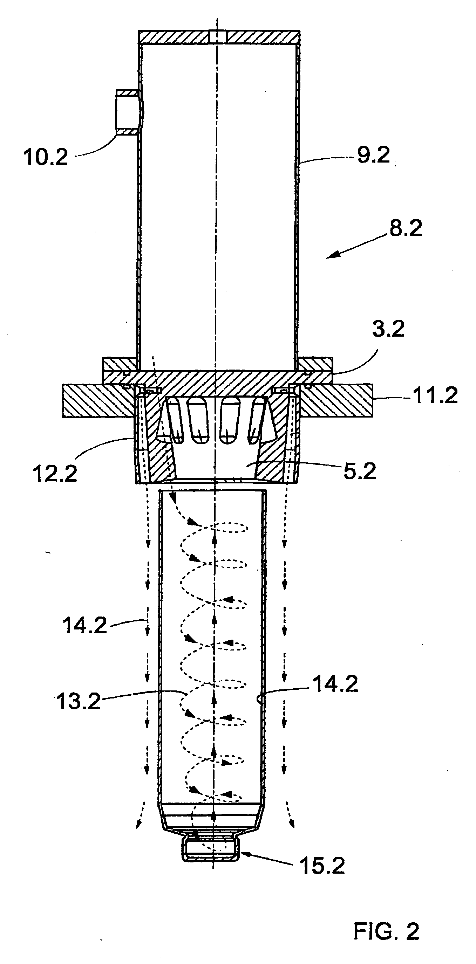

Fig. 2 shows the nozzle assembly 8.2 as a central cross section in a position corresponding to that which the assembly 8.2 may have when it is used in a filling machine. Supply with gas of the desired type (hot air, sterilization gas or sterile air or combinations thereof) takes place continuously at the central inflow connection 10.2. As the supplied gas first fills the inflow chamber 9.2, the flow which is fed to the package 1.2 via channels (not shown) will be able to maintain a uniform and constant pressure, which must be considered as a precondition for the function of the assembly 8.2 to maintain a continuous gas mass flow. The channels are obliquely inclined in the above described manner thereby gives rise to a helical gas mass flow 13.2 along the inner periphery of the package. Examples of inclinations are 0° radially and 14° tangentially, yet the invention is not presently limited in this respect, i.e. 0-5° radially and 0-20° tangentially is another example of intervals. When the gas mass flow reaches the bottom 15.2 of the package 1.2, it will, as a consequence of the lower gas pressure in the centre of the package, strive to leave the package in this section. Thus, the return flow of the supplied gas out of the package also takes place in a controlled manner. When the return flow reaches the opening of the package, it is taken care of in the return channel 5.2. In the upper region of the return channel this is deflected approx. 180° in order to be led out via the outer periphery of the package. There will thereby be created a downwardly directed flow stratum 14.2 along the circumferential surface of the package 1.2, which, when the gas consists of sterilization gas, sterilizes this surface and principally protects against re-infection. Since the velocity of the gas mass flow, as was mentioned by way of introduction, according to the invention is but a fraction of the previously employed velocity, the return flow flows as a boundary layer flow downwardly directed along the outside of the package. Further, the potential risk of re-infection of the package in the subsequent filling stage according to the prior art technology will be obviated since the constant sterile air flow which prevails there no longer runs the risk of being disturbed by the gas mass flows in the sterilization stage, the sensitivity is reduced by means of the increased flow rates.

For the purposes of the present invention the above described nozzle assembly have the advantageous feature of creating a downwardly directed flow on the outside of the package. The present invention should not, however, be construed as limited to this exact design of nozzle.

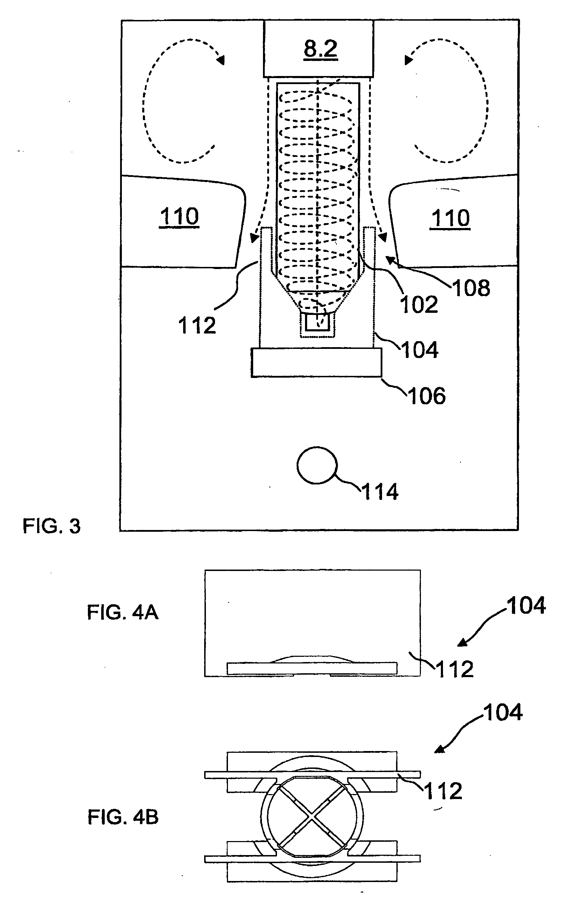

Fig. 3 illustrates a first embodiment of the invention, and represents a schematic cross section, orthogonal to the transportation direction of the packages 102 (i.e. in the machine direction), in the sterilizing zone of the filling machine. The package 102 is carried by a carrier 104 attached to a transportation line 106 (schematically represented), which is accommodated in the second volume. It should be noted that though the transportation line 106 is illustrated as a separate component below the carrier 104 its function may be realized by interconnecting adjacent carriers 104, such that these form a more direct part of the transportation line. In the first volume there is a need of an elevated concentration of sterilization agent, since gases or particles from this volume may have a chance of getting reintroduced into the interior of the package as the package is transported in the machine direction. The assembly 8.2 of Fig. 2 is arranged in the top of the first volume, and is used as a means for gas flow treatment (or gas injection means) of an open end of a package 102, as it injects a sterilization mixture into the package 102, as indicated by the dotted lines and arrows inside and just outside of the package 102. The first and second volume meet in a restriction zone, or venturi zone 108, having a smaller cross sectional area than the cross sectional area of the first and second volume surrounding it. The venturi zone is defined by structural indentations 110 extending in the machine direction of the channel. The flow pattern will follow the schematic indications of Fig. 2 and 3 when a package 102 is positioned below the nozzle assembly, and thus the mass flow in the surroundings of the package 102 will have a momentum component directed downwards, from the first volume to the second, and as it passes the venturi zone 108 the flow velocity or flow rate will increase, thus creating a more stable flow. The flow redirected in the nozzle assembly 8.2 will guide the flow outside the package and thus simultaneously forming of the gas barrier. Above the structural indentations 110 recirculation zones will be established, as indicated by the curved, dotted arrows.

In order to further direct and enhance the flow, flow directors 112 may arranged in connection to the venturi zone 108. These flow directors are preferably provided by a tailor-made design of the carriers 104, as shown in Figs. 4A and B. When a package is arranged in a carrier, the carrier's function as a flow director is limited, since it will be negligible compared to the effect of the package. Still, the carrier should be designed to interfere as little as possible with the flow, such that it does not support the generation of turbulent flows. If a package is not in arranged in the carrier its function as a flow director increases. Most surface area of the carrier is arranged in the vertical direction, so as to guide the flow downwards. When the carriers are indexed to a new position there may be a risk of generating swirls. In order to minimize this, the cross sectional area of the carrier in the direction orthogonal to the machine direction should be minimized. Any constructional portion extending in this direction is thus minimized, and arranged as far from the gas barrier as possible, i.e. as far down as possible, in order not to disturb the flow in the gas-barrier region. There may very well be slits between adjacent carriers, yet interlocking solutions are also feasible. As is visible in Fig. 3 the carrier extends into the venturi zone, and thus it assists in creating a further restriction of this zone and thus a more stable flow. The carriers may be provided with flow directors in the form of vertical planes for directing the flow downwards. The vertical planes preferably extend in the machine direction, in order to minimize the impact on the overall flow pattern during movement of the carrier. Gas evacuation means 114 are arranged in the lower volume. The gas evacuation means 114 may comprise a pipe extending in or across the machine direction of the channel, the pipe having openings for extraction of air distributed along its extension. A pipe extending in the cross direction will add to the constructional rigidity of the channel, and may therefore be the preferred solution of these two. The size of the openings as well as the evacuation rate may be balanced to support the creation of the air flow barrier.

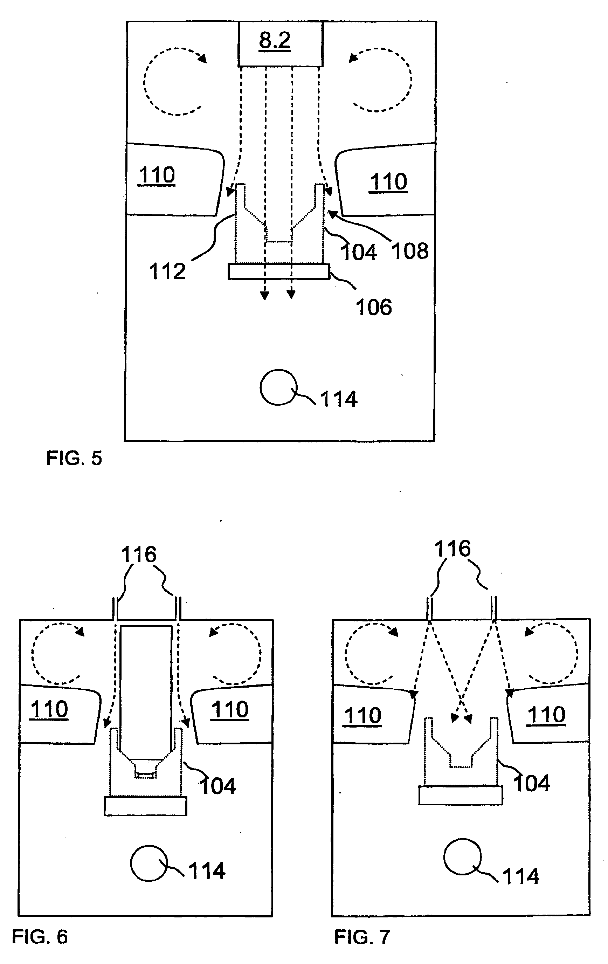

The presence of a package 102 will obviously affect the flow, the two major effects being that the radially inner component of the flow from the assembly 8.2 will be directed into the package 102 thus not reach the venturi zone unaffected, and that the package 102 will occupy part of the venturi zone 108. This in turn results in that the pressure difference between the upper and lower volume increases, to the benefit of the gas flow barrier. The general experience from simulations as well as experiments is that establishment of the gas barrier is not a problem when packages 102 are occupying the carriers. However, as schematically illustrated in Fig. 5, showing the same setup as Fig. 3 but without a package arranged in the carrier the inventive purposes are fulfilled without a package too. Figs 6 and 7 shows alternative applications of the inventive idea, this time in the filling zone of a filling machine, wherein the most fundamental difference as compared to the previous embodiments is the design and location of the nozzle assembly 116.

One part of the inventive idea is the insight that it is not necessary to create a global overpressure in the first, upper volume, or a global flow in the direction from the first volume towards the second, it suffices that the correct flow direction is arranged in an interface area between the first volume and the second. This insight results in several possibilities for optimization, as have been described above.

The present invention may be applied in a filling or packaging machine, further details of which are described in a number of copending Swedish patent applications, filed by the same applicant on the same day as the present application. To this end further details of:
A nozzle that may be used when treating the interior of the packaging containers is disclosed in the application with the title "A device and a method for gaseous-treatment of packages" (SE-0900909-9).
A device and method for maintaining asepticity is disclosed in "A device and a method for maintaining a gas flow barrier between two interconnected volumes" (SE-0900911-9).
A method for obtaining an optimized concentration of sterilization agent in a sterilization zone is disclosed in the application with the title "A device and a method for sterilizing packages" ((SE-0900907-7).
A system for ensuring that entrainment air is present for the jet flows of the filling zone and venting zone is disclosed in the application with the title "A system for treating packaging containers" (SE-0900912-7).
A device for providing cleaned air, which may be used for the as a source of entrainment air to jets in the venting zone and filling zone and surplus air in the filling zone, is disclosed in the application with the title "A device for cleaned air provision" (SE-0900908-5).

Some various aspect of the filling or packaging machine are disclosed in the applications titled "Packaging machine and packaging method I" (SE-0900909-3) and "Packaging machine and packaging method II" (SE-0900910-1), respectively. A system for supplying entrainment air to jet air flows in the machine are disclosed in the application with the title "A system for treating packaging containers" (SE-0900912-7).

## Claims

1. A device for maintaining, in a filling machine, a gas flow barrier between two volumes of a channel, said channel being adapted for transportation of packages (102) in a length direction thereof, and said volumes comprising a first volume having a first degree of sterilization, and a second volume having a second degree of sterilization, wherein
- the first volume comprises gas injection means (8.2, 116),
- the second volume comprises gas evacuation means (114),
- the first and second volume meet in an interface area extending in a length direction of the channel,
**characterized in that** the two volumes meet in venturi zone (108), a portion of the channel having a reduced cross section defined by structural indentations (110) extending in the length direction of the channel.

2. The device of claim 1, wherein the gas injection means comprise a nozzle (8.2, 116) arranged to direct a gas flow into a package and receive the flow returning from the package, and redirecting the flow essentially parallel to the outside of the package, in the direction from the first volume towards the second.

3. The device of any preceding claim, further comprising carrier means (104) for transportation of the packages by their closed end through the channel, wherein the carrier means comprise flow directors (112), for directing the gas flow.

4. The device of claim 3, wherein the flow directors extends into the venturi zone for reduction of the cross section of this zone.

5. A method for maintaining, in a filling machine, a gas flow barrier between two volumes of a channel, said channel being adapted for transportation of packages in a length direction thereof, and said volumes comprising a first volume having a first degree of sterilization, and a second volume having a second degree of sterilization, wherein
- the first volume comprises gas injection means,
- the second volume comprises gas evacuation means,
- the first and second volume meet in an interface area extending in a length direction of the channel,
**characterized in** the step of guiding the flow from the first volume to the second through a venturi zone, a portion of the channel having a reduced cross section, in which the two volumes meet, the reduced cross section being defined by structural by structural indentations extending in the length direction of the channel.

6. The method of claim 5, wherein the gas injection means comprise a nozzle, the method further comprising the step of directing a gas flow from the nozzle into a package, receiving the flow returning from the package with the nozzle, and redirecting, by means of the nozzle, the flow essentially parallel to the outside of the package, in the direction from the first volume towards the second.

7. The method of any one of claims 5-6, wherein carrier means are arranged for transportation of the packages by their closed end through the channel, wherein the carrier means comprise flow directors, for directing the gas flow.

8. The method of claim 7, wherein the flow directors extends into the venturi zone for reduction of the cross section of this zone.

## Patentansprüche

1. Vorrichtung zum Aufrechterhalten einer Gasflussbarriere zwischen zwei Volumen eines Kanals in einer Füllmaschine, wobei der Kanal zum Befördern von Verpackungen (102) in einer Längsrichtung davon ausgelegt ist und die Volumen ein erstes Volumen mit einem ersten Sterilisationsgrad und ein zweites Volumen mit einem zweiten Sterilisationsgrad umfassen, wobei
- das erste Volumen Gasinjektionsmittel (8.2, 116) umfasst,
- das zweite Volumen Gasevakuierungsmittel (114) umfasst,
- sich das erste und das zweite Volumen an einer Grenzfläche treffen, die sich in einer Längsrichtung des Kanals erstreckt,
**dadurch gekennzeichnet, dass** sich die beiden Volumen in einer Venturi-Zone (108) treffen, ein Abschnitt des Kanals einen verringerten Querschnitt aufweist, der durch strukturelle Einbuchtungen (110) definiert ist, die sich in der Längsrichtung des Kanals erstrecken.

2. Vorrichtung gemäß Anspruch 1, wobei die Gasinjektionsmittel eine Düse (8.2, 116) umfassen, die angeordnet ist, um einen Gasfluss in eine Verpackung zu lenken und den aus der Verpackung zurückkehrenden Fluss aufzunehmen und den Fluss im Wesentlichen parallel zu der Außenseite der Verpackung in die Richtung von dem ersten Volumen zu dem zweiten umzulenken.

3. Vorrichtung gemäß einem der vorstehenden Ansprüche, ferner umfassend Trägermittel (104) zum Befördern der Verpackungen mittels ihres geschlossenen Endes durch den Kanal, wobei die Trägermittel Flusslenker (112) zum Lenken des Flusses umfassen.

4. Vorrichtung gemäß Anspruch 3, wobei sich die Flusslenker in die Venturi-Zone zum Verringern des Querschnitts dieser Zone erstrecken.

5. Verfahren zum Aufrechterhalten einer Gasflussbarriere zwischen zwei Volumen eines Kanals in einer Füllmaschine, wobei der Kanal zum Befördern von Verpackungen in einer Längsrichtung davon ausgelegt ist und die Volumen ein erstes Volumen mit einem ersten Sterilisationsgrad und ein zweites Volumen mit einem zweiten Sterilisationsgrad umfassen, wobei
- das erste Volumen Gasinjektionsmittel umfasst,
- das zweite Volumen Gasevakuierungsmittel umfasst,
- sich das erste und das zweite Volumen an einer Grenzfläche treffen, die sich in einer Längsrichtung des Kanals erstreckt,
**gekennzeichnet durch** den der Schritt des Lenkens des Flusses von dem ersten Volumen zu dem zweiten durch eine Venturi-Zone, wobei ein Teil des Kanals einen verringerten Querschnitt aufweist, in dem sich die beiden Volumen treffen, wobei der verringerte Querschnitt **durch** strukturelle Einbuchtungen definiert ist, die sich in der Längsrichtung des Kanals erstrecken.

6. Verfahren gemäß Anspruch 5, wobei die Gasinjektionsmittel eine Düse umfassen, wobei das Verfahren ferner den Schritt des Lenkens eines Gasflusses von der Düse in eine Verpackung, Aufnehmen des aus der Verpackung zurückkehrenden Flusses mit der Düse und Umlenken des Flusses mithilfe der Düse im Wesentlichen parallel zu der Außenseite der Verpackung in die Richtung von dem ersten Volumen zu dem zweiten umfasst.

7. Vorrichtung gemäß einem der Ansprüche 5-6, wobei Trägermittel zum Befördern der Verpackungen mittels ihres geschlossenen Endes durch den Kanal angeordnet sind, wobei die Trägermittel Flusslenker zum Lenken des Flusses umfassen.

8. Vorrichtung gemäß Anspruch 7, wobei sich die Flusslenker in die Venturi-Zone zum Verringern des Querschnitts dieser Zone erstrecken.

## Revendications

1. Dispositif pour maintenir, dans une machine de remplissage, une barrière d'écoulement de gaz entre deux volumes d'un canal, ledit canal étant prévu pour transporter des paquets (102) dans une direction longitudinale de celui-ci, et lesdits volumes comprenant un premier volume ayant un premier degré de stérilisation, et un deuxième volume ayant un deuxième degré de stérilisation,
- le premier volume comprenant des moyens d'injection de gaz (8.2, 116),
- le deuxième volume comprenant des moyens d'évacuation de gaz (114),
- le premier et le deuxième volume se rejoignant au niveau d'une zone d'interface s'étendant dans une direction longitudinale du canal,
**caractérisé en ce que** les deux volumes se rejoignent au niveau d'une zone venturi (108), une portion du canal ayant une section transversale réduite définie par des indentations structurelles (110) s'étendant dans la direction longitudinale du canal.

2. Dispositif selon la revendication 1, dans lequel les moyens d'injection de gaz comprennent une buse (8.2, 116) prévue pour orienter un écoulement de gaz dans un paquet et recevoir l'écoulement revenant du paquet, et pour rediriger l'écoulement essentiellement parallèlement vers l'extérieur du paquet dans la direction allant du premier volume vers le deuxième.

3. Dispositif selon l'une quelconque des revendications précédentes, comprenant en outre des moyens porteurs (104) pour transporter les paquets par leur extrémité fermée à travers le canal, les moyens porteurs comprenant des éléments directeurs d'écoulement (112) pour diriger l'écoulement de gaz.

4. Dispositif selon la revendication 3, dans lequel les éléments directeurs d'écoulement s'étendent dans la zone venturi en vue de réduire la section transversale de cette zone.

5. Procédé pour maintenir, dans une machine de remplissage, une barrière d'écoulement de gaz entre deux volumes d'un canal, ledit canal étant prévu pour transporter des paquets dans une direction longitudinale de celui-ci, et lesdits volumes comprenant un premier volume ayant un premier degré de stérilisation, et un deuxième volume ayant un deuxième degré de stérilisation,
- le premier volume comprenant des moyens d'injection de gaz,
- le deuxième volume comprenant des moyens d'évacuation de gaz,
- le premier et le deuxième volume se rejoignant au niveau d'une zone d'interface s'étendant dans une direction longitudinale du canal,
**caractérisé par** l'étape consistant à guider l'écoulement depuis le premier volume jusqu'au deuxième à travers une zone venturi, une portion du canal ayant une section transversale réduite dans laquelle se rejoignent les deux volumes, la section transversale réduite étant définie par des indentations structurelles s'étendant dans la direction longitudinale du canal.

6. Procédé selon la revendication 5, dans lequel les moyens d'injection de gaz comprennent une buse, le procédé comprenant en outre l'étape consistant à diriger un écoulement de gaz depuis la buse dans un paquet, à recevoir l'écoulement revenant du paquet avec la buse, et à rediriger, au moyen de la buse, l'écoulement essentiellement parallèlement à l'extérieur du paquet, dans la direction allant du premier volume vers le deuxième.

7. Procédé selon l'une quelconque des revendications 5 à 6, dans lequel des moyens porteurs sont prévus pour transporter les paquets par leur extrémité fermée à travers le canal, les moyens porteurs comprenant des éléments directeurs d'écoulement pour diriger l'écoulement de gaz.

8. Procédé selon la revendication 7, dans lequel les éléments directeurs d'écoulement s'étendent dans la zone venturi en vue de réduire la section transversale de cette zone.
